# EUROPÄISCHE PATENTANMELDUNG

(11) **EP 4 180 047 A1**
(43) Veröffentlichungstag der Anmeldung: **17.05.2023**
(21) Anmeldenummer: 20944522.0
(22) Anmeldetag: 10.07.2020
(51) Int. Cl.: A61K 33/00, A61K 35/10, G01N 33/50

(54) **VERFAHREN ZUR DURCHFÜHRUNG EINES FASTENVERLAUFS FÜR SCHWERKRANKE PATIENTEN**

(71) Anmelder: Martynova, Elena Anatol'evna, Kiev, 03124 (UA)
(72) Erfinder: Martynova, Elena Anatol'evna, Kiev, 03124 (UA)
(74) Vertreter: Yefremenko, Olga
(86) Internationale Anmeldenummer: PCT/IB2020/000780
(87) Internationale Veröffentlichungsnummer: WO 2022/008943

(57) **Zusammenfassung**

Die Erfindung bezieht sich auf die Medizin und kann bei schwerkranken Patienten eingesetzt werden, die sich einem Fasten unterziehen, um ihre Schmerzen zu lindern. Bei dieser Methode ist die Nahrungs- und Flüssigkeitsaufnahme für mehrere Tage ausgeschlossen, und vor Beginn der Fastenkur wird eine vegetarische Diät eingehalten. Vorab werden dem Patienten lebende E.coli-Stämme typischer und laktosonegativer E.coli entnommen und unter künstlichen Bedingungen gezüchtet und während der gesamten Fastenzeit aufbewahrt. Während der abschließenden Diät werden die Kulturen des Patientenstammes typischer und laktosonegativer E.coli dem Patienten verabreicht; nach der ersten Darmentleerung wird die E.coli-Konzentration getestet und auf Übereinstimmung mit den Normen überprüft, wobei folgende Werte als normal gelten: typische E.E. coli 107-108 KBE/g Kot, E. coli laktosonegativ <105 KBE/g Kot, E. coli hämolytisch - 0. Bei Abweichung von der Norm mit unzureichenden E.coli-Werten wird eine zweite Verabreichung der entsprechenden typischen und/oder laktosonegativen E. coli. E.coli-Stamm des Patienten verordnet. Bei überhöhten E.coli-Werten werden die entsprechenden typischen und/oder laktosonegativen E.coli von der jeweiligen nächsten Mahlzeit ausgeschlossen. Bei hämolytischen E.coli wird eine orale Rehydratation verordnet.

## Beschreibung

Die Erfindung bezieht sich auf die Medizin und kann bei schwer kranken Patienten eingesetzt werden, die sich einem Fasten unterziehen, um ihre Schmerzen zu lindern.

Der moderne Mensch, der mehr als 4000 Chemikalien ausgesetzt ist, führt oft einen unnatürlichen Lebensstil, der von einer ungesunden Ernährung begleitet wird. In Kombination mit anderen Einflüssen führt dies häufig zu einer Störung der menschlichen Selbstregulierung, zu Veränderungen des Bewusstseins und zum Ausbruch verschiedener schwerer Krankheiten, einschließlich Krebs. Nach Angaben der International Union Against Cancer (1982) dauert die Induktionsphase des Krebses 15-20 Jahre, die In-situ-Phase - 5-10 Jahre, die Invasionsphase - 1-5 Jahre, die Disseminationsphase - 1-5 Jahre. Es ist erwiesen, dass in der In-situ-Phase durch Immunmechanismen reversible Veränderungen einer malignen, mutierten, renegierten Zelle möglich sind. Daraus folgt, dass jeder Einzelne nur 15-20 Jahre Zeit hat, um routinemäßig an der Primär- und Sekundärprävention von Krebs zu arbeiten.

Eine weitere Bedrohung sind heute Infektionskrankheiten wie Hepatitis, Tuberkulose, AIDS sowie schwere somatische Krankheiten wie Prostatitis, Myokarditis usw.

Die Suche nach Möglichkeiten, die gestörte Selbstregulation des menschlichen Körpers wiederherzustellen, war noch nie so dringlich wie heute, da viele der bestehenden wirksamen Methoden zur Beseitigung der Symptome bestimmter Krankheiten nicht zur Gesundheit des Patienten als Ganzes beitragen.

Es gibt eine bestehende Methode zur Behandlung onkologischer Erkrankungen, die die Einführung von Abkochungen pflanzlicher Arzneimittel und therapeutisches Fasten für einen Patienten umfasst (RU, Patent für die Erfindung Nr. 2074731, A61K 35/78, veröffentlicht am 10.03.1997). [1]).

Die bestehende Methode wird wie folgt umgesetzt: im Laufe des Tages mit einer allmählichen Erhöhung der Dosis wird Tinktur von Fruchtkörpern von rotem Fliegenpilz, WO 2022/008943PCT/IB2020/000780 Tinktur von Eisenhut-Wacholderwurzel, Tinktur von Schierlingsfleckenkraut und dann Abkochung von Sammlung 1 verabreicht, mit Schöllkraut, Cataranthus rosea und Mistel weiß, gefolgt von einer Abkochung der Sammlung 2 mit Schafgarbenkraut, Ringelblumenblüten und Löwenzahnwurzel sowie einer Tinktur aus Echinacea, Pallas's Distelwurzelpulver und Knoblauch alkoholische Tinktur, dann infundiert Brühe Sammlung 3, mit loquat Gras, Johanniskraut Kraut und Blumen Cypress schmalblättrigen, dann infundiert Propolis-Tinktur mit Honig, in regelmäßigen Abständen den ganzen Tag infundiert Pilz, und im Laufe der Behandlung regelmäßige Hungerbehandlung in Kombination mit Atemübungen, Kältebehandlungen, die Reinigung des Magen-Darm-Trakt. Das Fasten wird einmal alle 1-2 Wochen für zwei Tage durchgeführt.

Der Nachteil der bestehenden Methode ist die Verwendung von giftigen und allergenen Substanzen, was die Anwendung einer solchen Methode für eine Reihe von Patienten unmöglich macht. Die Methode ist nicht allgemeingültig und aufgrund der Verwendung von giftigen Substanzen recht gefährlich.

Die bestehende Methode der Rehabilitation des menschlichen Organismus, einschließlich des Hungerns und der diätetischen Ernährung, einschließlich der Eingangs- und Ausgangsphasen, ist aus dem Patent der Russischen Föderation Nr. 2028160, C1 (RU, Patent für Erfindung Nr. 2028160, C1, MPK6 A61M 21/00, veröffentlicht am 09.02.1995 [2]) bekannt. Bei dieser bestehenden Methode wird mit Hilfe der rationalen Psychotherapie zunächst das Wesen der Methode erklärt und die Möglichkeit der Heilung vorgeschlagen, dann werden Nahrung und Flüssigkeit für 7-11 Tage vollständig ausgeschlossen. Gleichzeitig wird der tägliche Biorhythmus des Schlafes in einen Wachrhythmus umgewandelt, so dass der Patient nachts wach und motorisch aktiv bleibt; es werden Kaltwasser-Luftanwendungen durchführt. Der Schlaf wird in den Morgen- und Abendstunden des Tages organisiert, während der Patient tagsüber wachgehalten wird, wobei das Sprechen einschränkt und plötzliche Bewegungen vermeidet werden.

Die bestehende Methode ermöglicht es, den Körper von Produkten des unvollständigen Stoffwechsels zu reinigen, den immunologischen Status des Körpers zu verbessern, die Widerstandskraft des Körpers zu erhöhen und Medikamentenbehandlung zu vermeiden.

Die bestehende Methode ist jedoch für die Patienten durch das Gefühl von unerträglichem Durst, das Auftreten erheblicher Schmerzen, insbesondere bei Krebspatienten, nur schwer zu tolerieren, was sie für eine Vielzahl schwer kranker Patienten inakzeptabel macht und keine Aktualisierung des Immunstatus ermöglicht. Darüber hinaus ist die bestehende Methode recht unbequem, da der tägliche Biorhythmus einer Person geändert werden muss, was einen negativen Einfluss auf die Genesung hat.

Im Allgemeinen besteht der Nachteil dieser Methode darin, dass es körperlich nicht oder nur sehr schwer möglich ist, eine vollständige Nahrungs- und Flüssigkeitsabstinenz bis zu 11 Tagen durchzuhalten und den Biorhythmus auf einen nächtlichen Wachmodus umzustellen, während sich der Patient gleichzeitig viel bewegt und Kaltwasser-Luft-Anwendungen durchführt, da die Änderung des Biorhythmus zu einem großen Feuchtigkeitsverlust führt. Es kommt zu einer schweren Dehydrierung mit einem hohen Sterberisiko. In diesem Zustand ist der Patient nicht in der Lage, ein aktives Leben zu führen oder irgendeine Arbeit zu verrichten, sondern kann nur ein bettlägeriges Leben führen.

Das ähnlichste Verfahren ist ein Verfahren zur Rehabilitation (RU2015106540, veröffentlicht am 20.09.2016), das einen Fastenkurs und eine diätetische Ernährung in den Eingangs- und Ausgangsstadien vorsieht, dadurch gekennzeichnet, dass als diätetische Ernährung eine vegetarische Ernährung verwendet wird und der Fastenkurs einen Grundzyklus mit ungeteilten, aufeinanderfolgenden Anfangs-, Haupt- und Endstadien umfasst. Dabei dauert das Anfangsstadium einen Tag lang, wobei Nahrung und Flüssigkeit, außer Wasser und Urin ausgeschlossen werden. Das Hauptstadium dauert mindestens 11 Tage mit vollständiger Ausschließung von Nahrung und Flüssigkeit. Das Abschlusstadium dauert einen Tag, wobei Nahrung und Flüssigkeit, außer Wasser und Urin ausgeschlossen werden. In der Hauptphase wird das Mundspülen vorgesehen. Eine Fastenkur umfasst zusätzlich mindestens einen zusätzlichen Fastenzyklus, der vor dem Hauptfastenzyklus oder vor einem anderen zusätzlichen Fastenzyklus durchgeführt wird, wobei der zusätzliche Fastenzyklus von dem Hauptfastenzyklus oder von einem anderen zusätzlichen Fastenzyklus durch eine vegetarische Diät getrennt wird, und jeder zusätzliche Fastenzyklus ungeteilt aufeinanderfolgende Anfangs-, Haupt- und Endphasen hat. Dabei dauert die Anfangsphase einen Tag mit Ausschluss von Nahrung und Flüssigkeit, außer Wasser und Urin. Die Hauptphase ist kürzer als 11 Tage und sieht einen vollständigen Nahrungs- und Flüssigkeitsverzicht vor. Die Endphase dauert einen Tag mit einem Verzicht auf Nahrung und Flüssigkeit außer Wasser und Urin. Während der Hauptphase jedes weiteren Fastenzyklus werden Mundspülungen verabreicht.

Das Vorgehen (RU2015106540, veröff.: 20.09.2016) wurde als Vorlage übernommen.
Die positiven Auswirkungen des Fastens sind auf die bekannten Auswirkungen des Fastens auf das Gehirn zurückzuführen: Verbesserung der kognitiven Funktionen, Erhöhung der neurotrophen Faktoren, Verbesserung der Stressresistenz, Abschwächung und Eliminierung von Entzündungen.
Hungern ist eine Herausforderung für das Gehirn, und das Gehirn nimmt diese Herausforderung an, indem es seine Reaktion auf Stress anpasst, was die anschließende Stressbewältigung erleichtert und die Wahrscheinlichkeit von Erkrankungen verringert.

So erhöht Fasten beispielsweise die Widerstandsfähigkeit gegen altersbedingte Erkrankungen. Bei der Parkinson-Krankheit bewahrt ein regelmäßiger Verzicht auf Nahrung die dopaminergen Neuronen und verringert das Fortschreiten der Krankheit. Bei der Alzheimer-Krankheit verhindert das Fasten die Anhäufung von fehlgefalteten Proteinen. Bei der Huntington-Krankheit normalisiert das Fasten den Spiegel des neurotrophen Faktors, der die neuronale Degeneration verlangsamt (Matson et al., 2008; Duan et al., 2003b). Ein- bis zweimal wöchentliches Fasten kann das Gehirn vor degenerativen Erkrankungen wie Parkinson und Alzheimer schützen, so eine Studie des National Institute on Ageing in Baltimore, USA. Hungern bekämpft die neuronale Pathologie, indem es den Gehalt an Proteinen erhöht, die die Anhäufung fehlgefalteter Proteine verhindern, ein Markenzeichen der Alzheimer- und Parkinson-Krankheit (Rubinsztein et al., 2011).

Außerdem steigert Hunger die Energieproduktion, indem er die Mitochondrien vor altersbedingten Schäden schützt (Liu et al., 2006).

Forschungen (Willette et al., 2012) haben gezeigt, dass Fasten das Gedächtnis, die Entscheidungsfindung und die emotionale Kontrolle verbessert, indem es das Volumen der an diesen Prozessen beteiligten Hirnstrukturen, einschließlich des Hippocampus, der Amygdala und des präfrontalen Kortex, bewahrt.

Darüber hinaus steigert Hungern die Neurogenese und stimuliert ihre trophischen Faktoren wie BDNF, insulinähnlicher Wachstumsfaktor 1 und vaskuläre endotheliale Wachstumsfaktorzellen (Lee et al., 2002; Cheng et al., 2003; Joseph D'Ercole und Sie, 2008; et al. Trejo et al., 2008).

In Studien (Harvey et al., 2011) reduzierte das Fasten Entzündungsmarker wie proinflammatorische Zytokine im Blut.

Hungern steigert auch die Durchblutung und die funktionelle Konnektivität in wichtigen Hirnregionen, einschließlich des Hippocampus und des anterioren cingulären Kortex (Burdett et al., 2010), erhöht die neuronale Konnektivität durch Steigerung der elektrischen und synaptischen Aktivität (Voss et al., 2010) und verhindert den Tod von Neuronen durch Induktion von Transkriptionsfaktoren, die für die Expression von Genen verantwortlich sind, die an der Regulierung des Zellüberlebens beteiligt sind (Mattson, 2008).

Hungern verlangsamt die zelluläre Spezifität des Alterns, die sich in der Anhäufung reaktiver Sauerstoffspezies ausdrückt, was sich in einer positiven Regulierung von Enzymen äußert, die oxidativen Stress hemmen (Hen et al., 2006), bewahrt die DNA-Integrität, indem es den altersbedingten Rückgang des DNA-Reparaturenzyms ARE1 abschwächt (Kisby et al., 2010), und stimuliert die Autophagie in Neuronen, was die Beseitigung beschädigter Proteine und toter Zellfragmente verbessert (Alirezaei et al., 2010).

So zeigt sich die wertvolle Wirkung des Fastens in der Unterbindung von Entzündungen und Schmerzen bei schwer kranken Menschen, wie z. B. Krebspatienten.

Die Anmelderin hat experimentell festgestellt, dass bei einer Hauptfastenkur, die aus drei ungeteilten Phasen besteht: einer Anfangsphase von 1 bis 3 Tagen mit Ausschluss von Nahrung und Flüssigkeit außer Wasser und Urin, einer Hauptphase von 11 Tagen oder mehr mit vollständigem Ausschluss von Nahrung und Flüssigkeit und einer Endphase von 1 bis 3 Tagen mit Ausschluss von Nahrung und Flüssigkeit außer Wasser und Urin sowie Mundspülung während der Hauptphase des Fastens, der Durst verringert wird und somit die Schmerzen der Patienten abnehmen.

Darüber hinaus werden durch das Hunger- und Durstgefühl der Patienten die mit verschiedenen Krankheiten verbundenen Schmerzen gemildert. Die Schmerzen werden erträglich, und die Patienten sind in der Lage, während des Fastens bestimmte Arbeiten auszuführen.

Bei Krebs wird die Schmerzempfindung durch den Tumor gedämpft, weil das Tumorwachstum aufgrund des Glukose- und Flüssigkeitsmangels im Blut des Patienten zum Stillstand kommt und die Erregung des Nervensystems, die mit dem Tumor zusammenhängt und Schmerzen verursacht, durch Nervenimpulse gedämpft wird, die aus dem Verdauungssystem kommen, hauptsächlich aus dem Magen, der Hunger und Durst verspürt. Auf diese Weise trägt die Verzweigung der Schmerzempfindungen dazu bei, die Aktivität der einzelnen Empfindungen zu dämpfen. D.h. einerseits hilft das Hungern, die Schmerzen der Krankheit zu verringern, und andererseits verringern die mit der Krankheit verbundenen Schmerzen die Unannehmlichkeiten des Hungerns und des Durstes und machen sie leichter erträglich.

Das Ergebnis ist eine Erneuerung des menschlichen Immunstatus für ein breites Spektrum von Patienten. Die in RU20 151 06540 vorgeschlagene Methode wirkt sich positiv auf die Durchblutungs- und Atmungsprozesse der menschlichen Haut aus und trägt zur Reduzierung von Übergewicht bei.

In diesem Zusammenhang führt die im Prototyp (RU2015106540) beschriebene Methode zur Rehabilitation des menschlichen Organismus, die eine Fastenkur und diätetische Mahlzeiten in der Eingangs- und Ausgangsphase umfasst, tatsächlich alle oben beschriebenen positiven Reaktionen auf den menschlichen Organismus aus.

Die prototypische Methode hat jedoch auch nicht realisierte Maßnahmen, die immer mit schwerkranken Menschen, wie z. B. Krebspatienten, einhergehen. Darunter versteht man den Zustand starker Schmerzen bei schwerkranken Patienten.

Selbstverständlich ist die Einnahme von Schmerzmitteln während des Fastens ausgeschlossen, da Schmerzmittel zu Vergiftungen und Leberfunktionsstörungen führen können.

Das technische Problem der Prototyp-Methode besteht also darin, dass sie die Frage der Schmerzlinderung nicht wirksam angeht. Daher können schwerkranke Patienten das Fasten manchmal nicht ertragen, weil sie unerträgliche Schmerzen haben.

Das Ziel der Erfindung ist es, das technische Problem des Prototyps zu beseitigen.

Das technische Ergebnis ist eine Linderung der Schmerzen bei den Patienten, wodurch sie das Fasten leichter ertragen können.

Das angegebene technische Ergebnis wird dadurch erreicht, dass eine Fastenmethode für schwerkranke Patienten beansprucht wird, bei der Nahrung und Flüssigkeit ausgeschlossen werden und vor Beginn einer Fastenkur von 5 bis 10 Tagen eine vegetarische Diät mit einer Diät aus Gemüse oder einer Gemüsediät in Kombination mit frisch gekochtem Getreide aus ganzen Getreidekörnern durchgeführt wird; das Fasten beginnt nach der Entleerung und umfasst einen Grundzyklus, der eine ungeteilte anschließende Basis-, Grund- und Endfastenphase umfasst, wobei die Basisphase über 1-3 Tage unter Ausschluss von Nahrung und Flüssigkeit außer Wasser und Urin durchgeführt wird, die Grundphase über mindestens 11 Tage unter Ausschluss von Nahrung und Flüssigkeit durchgeführt wird, und die Endphase über 1-3 Tage unter Ausschluss von Nahrung und Flüssigkeit außer Wasser und Urin durchgeführt wird, wobei in der Hauptphase eine Mundspülung erfolgt. ***Der Unterschied besteht darin,*** dass dem Patienten zuvor lebende E.coli-Stämme typischer und laktose-negativer E.coli entnommen und unter künstlichen Bedingungen gezüchtet werden; diese werden während der gesamten Fastenzeit aufbewahrt und dann während der vegetarischen Ernährung nach der letzten Fastenphase als gezüchtete Kulturen typischer und laktose-negativer E.coli-Stämme dem Patienten wieder gegeben; nach dem ersten Stuhlgang wird der Patient getestet und die E. coli-Konzentration auf Einhaltung der Normen überprüft, wobei die Norm wie folgt lautet: typische E. coli 10⁷ -10⁸ KBE/g fäkal, E. coli 10 -10 -10 E. coli laktosonegativ < 10⁵ CFU/g Faeces, E. coli hämolytisch - 0; und im Falle einer Abweichung von der Norm mit unzureichenden E. coli-Werten, eine zweite Einnahme eines geeigneten typischen und/oder laktosonegativen E. coli-Stammes des Patienten, und bei einem Überschuss an E.coli, werden die entsprechenden typischen und/oder laktosonegativen E.coli bei der nächsten Mahlzeit ausgeschlossen; werden hämolytische E.coli nachgewiesen, wird eine orale Rehydratation verordnet, wobei der Patient bis zu 3 Liter Flüssigkeit pro Tag trinken sollte.

Es ist akzeptabel, dass am Ende einer Fastenkur eine vegetarische Diät mit einer Dauer von 10 Tagen durchgeführt wird, die eine pflanzliche Diät oder eine pflanzliche Diät in Kombination mit frisch zubereiteten Breien aus ganzen Getreidekörnern ist, und bei Patienten mit Anzeichen von Dystrophie eine vegetarische Diät mit einer Dauer von bis zu 14 Tagen.

Es ist akzeptabel, kaliumreiche Lebensmittel zu essen.

Zwischen den Fastenkursen können drei Wochen lang 250 g Kaogsil eingenommen werden.

Es ist akzeptabel, die Hauptphase des Basenfasten-Zyklus 22 Tage lang durchzuführen.

Es ist akzeptabel, dass ein Fastenzyklus einen weiteren Fastenzyklus einschließt, der vom Hauptfastenzyklus bzw. von einem anderen weiteren Fastenzyklus durch eine vegetarische Diät getrennt wird. Jeder weitere Fastenzyklus hat eine ungeteilte, aufeinander folgende Anfangs-, Haupt- und Endphase, wobei die Anfangsphase 1 bis 3 Tage ohne Nahrung oder Flüssigkeit außer Wasser und Urin dauert, die Hauptphase weniger als 11 Tage mit vollständigem Nahrungs- und Flüssigkeitsausschluss dauert und die Endphase 1 bis 3 Tage ohne Nahrung oder Flüssigkeit außer Wasser und Urin dauert. In der Hauptphase jedes weiteren Fastenzyklus erfolgt Mundspülung.

Es ist akzeptabel, während des Fastens mit Wasser oder leichten Getränken zu gurgeln.

Es ist akzeptabel, dass die Dauer einer vegetarischen Diät zwischen zwei zusätzlichen Fastenzyklen oder zwischen einem zusätzlichen Fastenzyklus und dem Hauptfastenzyklus ein bis zehn Tage eingehalten wird, wobei die Dauer der vegetarischen Diät zwischen zwei zusätzlichen Fastenzyklen der Dauer der Hauptfastenphase des vorangegangenen zusätzlichen Fastenzyklus entspricht.

Es ist akzeptabel, weiße Tonerde nach einer Fastenkur vor dem Hintergrund einer Diät einzunehmen.

### Ausführung der Erfindung

Gemäß der Industrienorm 91500.1 1.0004-2003, Patientenmanagementprotokoll.

Dysbakteriose des Darms", VERABSCHIEDET durch den Erlass des Gesundheitsministeriums der Russischen Föderation vom 09.06.2003 Nr. 231, soll die qualitative und quantitative Zusammensetzung der grundlegenden Mikroflora des Dickdarms bei gesunden Menschen E.coli typisch 10⁷ - 10⁸ KBE/g fäkal, E.coli laktosonegativ < 10⁵ KBE/g fäkal, E.coli hämolytisch - 0 betragen.

Abweichungen von diesen Werten sind ein Zeichen für eine Dysbiose:
- die Verringerung der typischen E. coli auf 10⁵ -10⁶ KBE/g oder eine Zunahme der typischen Escherichia coli auf 10⁹ -10¹⁰; KBE/r wird als erster Grad der mikrobiologischen Störungen definiert;
- der Anstieg der Konzentration von hämolytischen Escherichia coli auf bis zu 10⁵ -10⁷ KBE/g wird als mikrobiologische Störung zweiten Grades definiert.

E. coli sind die Hauptkonkurrenten der opportunistischen Mikroflora bei der Besiedlung des Darms. E. coli nehmen Sauerstoff aus dem Darmlumen auf, der für die nützlichen Bifidobakterien und Laktobazillen schädlich ist. E. coli produzieren eine Reihe von Vitaminen, die für den Menschen notwendig sind: B1, B2, BZ, B5, B6, Biotin, B9, B12, K, Fettsäuren (Essig-, Ameisen- und - einige Stämme - auch Milch-, Bernsteinsäure und andere Säuren), beteiligt sich am Stoffwechsel von Cholesterin, Bilirubin, Cholin, Gallensäuren, beeinflusst die Aufnahme von Eisen und Kalzium.

Bei normalen, gesunden Patienten, die nur eine Dysbakteriose haben, werden bei übermäßigem E. coli-Wachstum die folgenden Bakteriophagen empfohlen (je nach E. coli-Typ): Coli-Bakteriophage flüssig, Coliprotein-Bakteriophage flüssig, Pyobakteriophage kombiniert flüssig, Pyopolyphage in Tabletten, Pyobakteriophage polyvalent gereinigt flüssig oder Intesti-Bakteriophage flüssig.

Bei schwer kranken Menschen, wie z. B. Krebspatienten, die fasten, um ihre Schmerzen zu lindern, sind diese traditionellen Methoden jedoch aus verschiedenen Gründen nicht anwendbar.

Der Fastenzustand selbst verursacht starke Beschwerden im gesamten Darm, verbunden mit starken Blähungen während der Fütterungszeiten aufgrund der während der Fastenzeit entwickelten Dysbakteriose, die durch das Absterben der Bakterien, die die E.coli-Population regulieren, ohne Nahrung im Darmtrakt verursacht wird.

Dieser Zustand des Unbehagens verstärkt die ohnehin schon starken Schmerzen der schwer kranken Patienten.

Die beanspruchte Erfindung ermöglicht es, die Schmerzen bei Fastenkuren zu verringern und diese leichter zu ertragen.

Die Methodik der beanspruchten Erfindung basiert auf der Regulierung von E.coli-Populationen bei kritisch kranken Patienten.

Aus den Fäkalien oder dem Urin des Patienten werden zunächst lebende Stämme typischer E. coli und laktose-negativer E. coli entnommen und in Kulturen unter künstlichen Bedingungen gezüchtet.

Die Fäkalien werden in einem Konservierungsmittel gesammelt, bei dem es sich um phosphatgepufferte Kochsalzlösung oder Glycerinmischungen handeln kann. Wenn das Material innerhalb von 2 Stunden nach der Entnahme geliefert wird, werden die nativen Fäkalien in sterilen Fläschchen gesammelt. Nach der äußeren Genitaltoilette wird der Urin in sterilen Gefäßen gesammelt. Der Urin wird zentrifugiert, und das Sediment wird vor der Inokulation beimpft. Im Falle einer geringen Menge an Präzipitat wird nativer Urin in doppelter Konzentration in Anreicherungsmedium in gleichen Volumina beimpft.

Wenn die Menge des Erregers in den Proben gering ist und es nicht möglich ist, ihn mit der klassischen Methode zu isolieren oder ein schnelles Ergebnis zu erhalten, ist es möglich, E.coli direkt aus den Fäkalien zu bestimmen.

Der Nachweis von E. coli in den Fäkalien von Patienten ist ebenso zuverlässig wie die Isolierung des Erregers. Die wichtigsten Vorteile dieser Methode sind der schnelle Nachweis (da keine Isolierung von Reinkulturen erforderlich ist) und die Möglichkeit, ihre Konzentration in verschiedenen Stadien der Erkrankung zu bestimmen.

Der Gehalt an typischen, laktose-negativen und hämolytischen E. coli wird von bakteriologischen Labors bestimmt.

Die kultivierten typischen und laktose-negativen E.coli-Stämme werden während der gesamten Fastenzeit aufbewahrt, und während der Fütterungszeiten des Patienten werden die Kulturen der typischen und laktose-negativen E.coli-Stämme dem Patienten verabreicht.

Nach dem ersten Stuhlgang wird der Patient getestet und die E.coli-Werte werden auf Einhaltung der Normen überprüft, wobei die Norm wie folgt lautet: typische E.coli 10⁷ - 10⁸ KBE/g Stuhl, E.coli laktosonegativ < 10⁵ KBE/g Stuhl, E.coli hämolytisch - 0. Bei anormalen E.coli-Werten wird der entsprechende typische und/oder laktose-negative E.coli-Stamm erneut verabreicht, und bei übermäßigen E.coli-Werten wird der entsprechende typische und/oder laktose-negative E.coli-Stamm bei der nächsten Mahlzeit ausgeschlossen. Wenn hämolytische E.coli gefunden werden, wird eine orale Rehydratation verordnet, bei der der Patient mindestens 3 Liter Flüssigkeit pro Tag trinken sollte.

Ein wichtiges Merkmal ist die Verwendung eigener typischer und laktosonegativer E.coli-Stämme des Patienten, da die Verwendung von Darreichungsformen von E.coli-Stämmen zu einem unkontrollierten Wachstum typischer und laktosonegativer E.coli und anderer Stämme führen könnte, deren Wachstum ein hungernder Organismus nicht kontrollieren kann, insbesondere bei Dysbakteriosen. Dies ist darauf zurückzuführen, dass zahlreiche Pathogenitätsfaktoren bekannt sind: Adhäsion und Kolonisierung, Invasion, Exotoxine in pathogenen E.coli, Hämolysine, Shiga-Toxin (STX), Toxin L (thermolabiles Toxin), Toxin ST (thermostabiles Toxin), CNF (zytotoxischer nekrototoxischer Faktor), CLTD-Toxin, EAST-Toxin, Endotoxine Lipopolysaccharide.

Die Pathogenitätsfaktoren von E. coli werden nicht nur von den chromosomalen Genen der Wirtszelle kontrolliert, sondern auch von Genen, die durch Plasmide oder moderat konvertierende Phagen eingeführt werden. All dies deutet daraufhin, dass pathogene Varianten von E. coli durch die Verbreitung von Plasmiden und moderaten Phagen unter ihnen entstehen können. Die Pathogenitätsfaktoren werden bei hungernden Menschen verstärkt.

Infolgedessen besteht bei fastenden Patienten ein hohes Risiko für eine übermäßige Zunahme typischer und laktose-negativer E. coli, so dass sogar hämolytische E. coli-Bakterien nachgewiesen wurden. Infolgedessen muss der Patient das Fasten beenden und mit der Einnahme von Fluorchinolonen beginnen.

Es wurde experimentell festgestellt, dass die Verwendung typischer und laktosonegativer E.coli-Stämme bei der Aufnahme von patienteneigenen E.coli nur selten zu einer überdurchschnittlichen Zunahme der E.coli führt, und selbst dann nur geringfügig. Diese Geringfügigkeit ist so, dass es zur Angleichung der E.coli-Zahlen an den Normalwert ausreichte, die Aufnahme der entsprechenden typischen und/oder laktose-negativen E.coli bei der nächsten Mahlzeit auszuschließen. Und wenn die E.coli-Zahlen den Normalbereich überschritten, war der Anstieg der hämolytischen E.coli so gering, dass es nicht notwendig war, die Fastenkur abzubrechen. Die Verschreibung einer oralen Rehydratation, bei der der Patient mindestens 3 Liter Flüssigkeit pro Tag trinken sollte, erwies sich als ausreichend. Dies half, das Wasser-Salz-Gleichgewicht wiederherzustellen und den Flüssigkeitsverlust im Körper auszugleichen. Zur oralen Rehydratation kann jedes Rehydratationsmedikament verwendet werden, das als Pulver zur Herstellung einer Lösung erhältlich ist.

Die Umsetzung der Methode zur Rehabilitation des menschlichen Körpers beginnt mit der Umstellung auf eine vegetarische Ernährung, deren Grundprinzipien darin bestehen, auf Lebensmittel tierischen Ursprungs zu verzichten und Proteine, Enzyme, Vitamine, Mineralien und Aminosäuren aus pflanzlichen oder lakto-vegetarischen Lebensmitteln zu gewinnen. Insbesondere eine pflanzliche Ernährung oder eine pflanzliche Ernährung in Kombination mit Brei aus frisch gekochten Getreidevollkornprodukten. Eine solche Diät sollte 5-10 Tage lang eingehalten werden. Nach einer physiologischen Reinigung (Entleerung) wird mit einer Fastenkur begonnen. Für Patienten, die bereits eine Fastenkur gemacht haben und wissen, wie man sie durchführt, enthält die Fastenkur in der Regel nur den Hauptzyklus des Fastens, der ungeteilte aufeinanderfolgende Phasen umfasst: Anfangs-, Haupt- und Endphase. Zunächst für ein bis drei Tage auf Nahrung und Wasser verzichten, aber Wasser und gegebenenfalls Urin trinken (Anfangsphase), dann für 11 Tage oder länger auf jegliche Nahrung und Flüssigkeit verzichten (Hauptphase), und dann für ein bis drei Tage auf Nahrung und Flüssigkeit verzichten, aber Wasser und gegebenenfalls Urin trinken (Endphase). In der Hauptphase des Hauptfastenzyklus wird eine Mundspülung durchgeführt. Wasser, auch kühles Wasser, ist das am besten geeignete Mundwasser. Andere flüssige Lebensmittel, insbesondere Getränke, können ebenfalls verwendet werden. Allerdings sollten keine dunklen oder bunten Flüssigkeiten oder Tinkturen von Heilpflanzen verwendet werden, um den Zahnschmelz nicht zu beschädigen. Wasser (Flüssigkeit) sollte nach der Mundspülung gründlich ausgespuckt werden. Eine Fastenkur wird vor dem Hintergrund von Selbstkonditionierung und kontrollierter Vorstellungskraft durchgeführt, um Heilung zu erreichen. Zusätzlich können während der Hauptfastenphase des Hauptfastenzyklus Kältebehandlungen (Güsse) durchgeführt werden.

Darüber hinaus können während des Fastens Atemübungen, körperliche Übungen und eine Reihe von physiologischen Verfahren durchgeführt werden, die je nach Krankheit und Zustand des Patienten empfohlen werden.

Für Patienten, die zum ersten Mal fasten, beginnt die Umsetzung der Rehabilitationsmethode des Körpers auch mit der Einhaltung einer vegetarischen Diät mit Diätbeschränkungen, insbesondere einer pflanzlichen oder veganen Ernährung in Kombination mit frisch zubereiteten Breien aus Vollkorngetreide. Diese Diät sollte 10 Tage lang eingehalten werden. Nach der physiologischen Reinigung (Entleerung) wird eine Fastenkur eingeleitet, die mindestens einen weiteren Fastenzyklus umfasst, wobei der Hauptfastenzyklus der letzte ist.

Jeder zusätzliche Fastenzyklus wird nach Bedarf durchgeführt und unterscheidet sich vom Hauptfastenzyklus nur durch die Dauer der Hauptfastenphase, die im zusätzlichen Zyklus weniger als 11 Tage beträgt.

Patienten, die zum ersten Mal an einer Fastenkur teilnehmen, führen daher ein Kaskadenfasten durch: 1 bis 10 zusätzliche Zyklen, die jeweils einen Tag der Nahrungs- und Flüssigkeitsabstinenz außer Wasser und, falls gewünscht und nicht kontraindiziert, Urin (Anfangsphase), dann einen Tag bis 8-11 Tage ( Mindestdauer) der Nahrungs- und Wasserabstinenz (Hauptphase), jedoch mit Mundspülung, und dann einen bis einen Tag der Nahrungs- und Flüssigkeitsabstinenz außer Wasser und, falls gewünscht und nicht kontraindiziert, Urin (Endphase) umfassen. Anschließend wird der Patient ein bis zehn Tage lang vegetarisch ernährt und erhält eine Diät. Die Dauer der vegetarischen Ernährung kann der Dauer der Hauptphase des vorangegangenen zusätzlichen Fastenzyklus entsprechen. Dann beginnt die folgende Kaskade: ein zusätzlicher Fastenzyklus mit ein- bis dreitägiger Abstinenz von Nahrung und anderen Flüssigkeiten als Wasser und Urin (Anfangsphase), dann ein- bis zehntägige Abstinenz von jeglicher Nahrung und Wasser (Hauptphase), jedoch unter Verwendung von Mundspülungen, und anschließend ein- bis eintägige Abstinenz von Nahrung und anderen Flüssigkeiten als Wasser und Urin (Endphase), oder Durchführung des Hauptzyklus wie oben beschrieben.

Eine erste Fastenkur kann beispielsweise mit bis zu 10 zusätzlichen Fastenzyklen durchgeführt werden, wobei die Hauptfastenphase des zusätzlichen Fastenzyklus schrittweise von einem auf 10 Tage bzw. von einem auf 10 Tage vegetarische Ernährung mit einer Diät zwischen den zusätzlichen Zyklen und zwischen dem zusätzlichen und dem Hauptfastenzyklus erhöht wird.

Während der Hauptfastenphase jedes Ergänzungszyklus und der Hauptfastenphase des Hauptzyklus kann Wasser für Erkältungsbehandlungen (Güsse) und für Mundwasser verwendet werden. Das Wasser sollte nach der Mundspülung gründlich ausgespuckt werden. Der Fastenkurs wird vor dem Hintergrund von Selbstkonditionierung und geführter Imagination durchgeführt, um Heilung zu erreichen.

So enthält der Fastenkurs nur einen einzigen Fastenzyklus, der durch eine vollständige Abstinenz von Nahrung und Flüssigkeit über einen Zeitraum von 11 Tagen und mehr (bis zu 22 Tagen) gekennzeichnet ist und den grundlegenden und obligatorischen Zyklus des Fastens darstellt. Im Gegensatz zur Prototyp-Methode, die von den Patienten praktisch nicht toleriert werden kann, es sei denn, sie legen sich hin, wird bei der beanspruchten Methode zunächst eine eintägige Fastenphase mit Ausschluss von Nahrung und Flüssigkeit außer Wasser und Urin durchgeführt, bevor die vollständige Nahrungs- und Flüssigkeitsabstinenz über einen Zeitraum von 11 Tagen folgt. In der Hauptphase über einen Zeitraum von 11 Tagen oder mehr werden während der Hauptphase des Fastens Mundspülungen durchgeführt. Darüber hinaus erfolgt der Rückzug aus der Hauptphase ebenfalls schrittweise für 1 Tag mit dem Verzicht auf Nahrung und Flüssigkeiten außer Wasser und Urin. Diese Maßnahmen verringern das Durstgefühl des Patienten im Vergleich zur Prototyp-Methode. Das Ergebnis ist eine Erleichterung ihres Zustands, wodurch sich die Schmerzen der Patienten lindern.

Nach dem Fasten erfolgt eine vegetarische Diät mit Ernährungseinschränkung für 5 bis 10 Tage bzw. bis zu 14 Tage bei Patienten mit Anzeichen von Dystrophie, wobei der Patient durch die Einnahme von weißem Ton und/oder Urin in eine vegetarische Diät mit Ernährungseinschränkung aufgenommen wird.

Während einer Fastenkur treten auch unangenehme Empfindungen wie Hunger, Schwindel, Schmerzen, Krämpfe, Schwäche, Reizbarkeit, schlechte Laune, Übelkeit und Erbrechen auf, allerdings in geringerem Ausmaß und weniger häufig. Sie können diese unangenehmen Empfindungen auch durch Selbstverabreichung, Mundspülung, Atemübungen, körperliche Übungen, Selbstmassage, Massage und andere physiologische Verfahren, einschließlich der chinesischen Medizin, bekämpfen. Darüber hinaus sind frische Luft und kalte Duschen hilfreich. Zu jeder Jahreszeit sollte man an der frischen Luft spazieren gehen, wenn möglich mit entblößten Körperteilen. Es ist auch möglich, den Zustand durch längeres moderates Joggen, Atemanhalten, japanisches Reiki (Energie zum Heilen erhalten), Meditation, mentale Kontrolle der Vorstellungskraft, Selbstermutigung zu verbessern.

Bei schweren und vernachlässigten Formen der Krankheit kann die vorgeschlagene Methode periodisch und regelmäßig bis zur Selbstheilung durchgeführt werden, wobei zwischendurch die Homöopathie eingesetzt werden sollte. Nach vollständiger Genesung sollte die Methode alle 6 Monate zur Prophylaxe durchgeführt werden.

Nicht geeignet ist die Methode bei psychischen Erkrankungen, Hämophilie, Zerebralparese, Nierenversagen und akuten Niereninfektionen. Die Methode ist nicht für schwangere Frauen geeignet, es sei denn, sie wissen, wie sie die natürliche Energie nutzen können. Die angegebene Methode ist bei Leberkrebs nicht angewandt worden, was jedoch nicht bedeutet, dass sie unmöglich oder unwirksam ist.

Die vorgeschlagene Methode wurde an einer Gruppe von 7 Patienten getestet, die ihr schriftliches Einverständnis zur Durchführung der Methode zur Rehabilitation ihres Körpers gaben. Die Methode wurde in einem an der Küste gelegenen Kurortkomplex angewandt.

Die nachstehenden Beispiele zeigen, wie der Körper des Patienten der oben genannten Gruppe realisiert wird.

### Beispiel 1.

Bei Patientin N., 41 Jahre alt, wurde Brustkrebs rechts im Stadium HA pT2N0M0 diagnostiziert. Der Tumor befand sich im zentralen Quadranten. Sie wurde ein Jahr lang an ihrem Wohnort behandelt, wo man ihr einen chirurgischen Eingriff anbot.

Das Aushungern wurde bisher noch nicht eingesetzt.

Nach den Ergebnissen der ersten Tests im biologischen Labor beträgt der Gehalt an typischen E.coli 10⁸ CFU/g Fäkalien, E.coli laktosonegativ < 4×10⁴ CFU/g Fäkalien, E.coli hämolytisch - 0.

Die typischen und laktose-negativen E.coli-Stämme des Patienten wurden zur Kultivierung entnommen. Während der Kultivierung wurden die E.coli des Patienten in Fläschchen mit physiologischer Lösung im Kühlschrank aufbewahrt.

Der erste Kurs des Patienten umfasste Fasten und Diät:
- Gemüsediät in Kombination mit Breien aus frisch gekochtem Vollkorngetreide -7 Tage;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung -10 Tage;

### I zusätzlicher Fastenzyklus:

- Anfangsphase -1 Tag;
- Hauptbühne - 3 Tage;
- Schlussphase -1 Tag;
- vegetarische Ernährung - 3 Tage;

### Zweiter zusätzlicher Fastenzyklus:

- Anfangsphase -1 Tag;
- Hauptbühne - B-Tage;
- Abschlussphase - 1 Tag;
- vegetarische Ernährung - 6 Tage;

### III zusätzlicher Fastenzyklus:

- Anfangsphase - 1 Tag;
- Hauptphase - 9 Tage;
- Schlussphase -1 Tag;
- vegetarische Ernährung - 9 Tage.

Während der Hauptphase jedes zusätzlichen Fastenzyklus und während der Hauptphase des Hauptfastenzyklus wurde Mundwasser verwendet, das die Patientin N. jedes Mal gründlich ausspuckte, sowie Wasser zum Übergießen. Mundspülungen wurden 20- bis 40-mal pro Tag durchgeführt, Güsse wurden 5-6-mal pro Tag verabreicht.

In der Anfangs- und Endphase des Haupt- und Zusatzfastenzyklus trank Patientin N. etwa 1,5 Liter Wasser pro Tag.

Während des gesamten Fastenverlaufs wurden regelmäßig Selbstkodierungen und geführte Imaginationen durchgeführt, um Heilung zu erreichen.

Während der gesamten Rehabilitationsphase wurden jeden Morgen Übungen, einschließlich Atemübungen nach der Yogamethode, tägliche Luft- und Wasserbehandlungen, moderate Massagen und abendliche Spaziergänge am Meer durchgeführt.

Patientin N. hat die erste Rehabilitationsmaßnahme gut überstanden und ihr Allgemeinzustand hat sich deutlich verbessert. Ihr Durstgefühl war mäßig und ihre Schmerzen waren erträglich.

Nach drei Monaten unterzog sich Patientin N. einer zweiten Rehabilitationskur nach folgendem Schema: 7 Tage lang vegetarische Ernährung; Einnahme von typischen E.coli, E.coli laktosonegativ nach jeder Mahlzeit. Typische und laktosonegative E. coli-Werte waren normal, keine hämolytischen.

### Verlauf des Fastens (nach der Entleerung)

Grundlegender Fastenzyklus:
- Anfangsphase -1 Tag;
- Hauptphase -11 Tage;
- Schlussphase -1 Tag;
- Gemüsediät - 10 Tage; Aufnahme von typischen E.coli, E.coli laktose-negativ nach jeder Mahlzeit

### I. zusätzlicher Fastenzyklus:

- Anfangsphase -1 Tag;
- Hauptphase - 5 Tage;
- Schlussphase -1 Tag;
- vegetarische Ernährung - 5 Tage; Aufnahme von typischen E.coli, E.coli laktose-negativ nach jeder Mahlzeit.

### II. zusätzlicher Fastenzyklus:

- Anfangsphase - 1 Tag;
- Hauptphase - 8 Tage;
- Abschlussphase - 1 Tag;
- vegetarische Ernährung - 8 Tage; Aufnahme von typischen E.coli, E.coli laktose-negativ nach jeder Mahlzeit.

Während der Hauptphase des Fastens eines jeden Ergänzungszyklus und während der Hauptphase des Fastens des Hauptzyklus wurde Mundwasser verwendet, das die Patientin N. jedes Mal gründlich ausspuckte, sowie Wasser zum Begießen. Mundspülungen wurden 20- bis 40-mal pro Tag durchgeführt, Güsse wurden 5-8-mal pro Tag verabreicht.

In der Anfangs- und Endphase des Haupt- und Zusatzfastenzyklus trank Patientin N. etwa 1,5 Liter Wasser pro Tag.

Während des gesamten Fastenverlaufs wurden regelmäßig Selbstkodierungen und geführte Imaginationen durchgeführt, um Heilung zu erreichen.

Während der gesamten Rehabilitationsphase wurden jeden Morgen Übungen, einschließlich Atemübungen nach der Yoga-Methode, tägliche Luft- und Wasserbehandlungen, moderate Massagen und abendliche Spaziergänge am Meer durchgeführt.

Patientin N. hat die zweite Rehabilitationsmaßnahme gut überstanden und ihr Allgemeinzustand hat sich deutlich verbessert. Ihr Durstgefühl war mäßig und ihre Schmerzen waren erträglich.

Nach 4 Monaten unterzog sich der Patient N. einer dritten Rehabilitationsmaßnahme nach demselben Schema;
- Fastenkurs (nach der Entleerung):
- der Grundzyklus des Fastens:
- Anfangsphase -1 Tag;
- Hauptphase -11 Tage;
- Schlussphase -1 Tag;
- Vegetarische Ernährung - 14 Tage, vegetarische Ernährung in Kombination mit Schilderbrei - 10 Tage; Einnahme von typischen E.coli, E.coli laktose-negativ nach jeder Mahlzeit.

Während der Hauptphase wurde Mundwasser verwendet, das Patientin N. jedes Mal gründlich ausspuckte, sowie Wasser zum Begießen. Die Mundspülung wurde 30-35 Mal pro Tag durchgeführt, das Abbrausen einmal pro Tag.

In der Anfangs- und Endphase des Hauptfastenzyklus trank die Patientin N. etwa 1,5 Liter Wasser pro Tag.

Während des gesamten Fastenverlaufs wurden regelmäßig Selbstkodierungen und geführte Imaginationen durchgeführt, um Heilung zu erreichen.

Während der gesamten Rehabilitationsphase wurden jeden Morgen Übungen, einschließlich Atemübungen nach der Yoga-Methode, tägliche Luft- und Wasserbehandlungen, moderate Massagen und abendliche Spaziergänge am Meer durchgeführt.

Patientin N. hat ihre dritte Rehabilitationsmaßnahme gut überstanden und ihr Allgemeinzustand hat sich verbessert. Die Ergebnisse der Diagnose zeigen eine Besserung ihres Zustands.

Es wird empfohlen, zweimal im Jahr eine Rehabilitationskur für den Körper nach derselben Methode durchzuführen.

Nach dem Fasten nahmen die Schmerzen ab und es gab keine Blähungen bei den Mahlzeiten. Die Patientin hat das Fasten gut vertragen.

### Beispiel 2.

Bei der Patientin O. wurde ein Zervixkarzinom im Stadium III diagnostiziert.
Ein Jahr lang sie ich an ihrem Wohnort behandelt, wo man ihr eine Operation vorschlug.

Das Aushungern wurde bisher noch nicht eingesetzt.

Nach den Ergebnissen der ersten Tests im biologischen Labor beträgt der Gehalt an typischen E.coli 6×10⁷ CFU/r Faeces, E.coli laktose-negativ < 6×10⁴ CFU/g Faeces, E.coli hämolytisch - 0.

Die typischen und laktose-negativen E.coli-Stämme der Patientin wurden zur Kultivierung entnommen. Während der Kultivierung wurden die E.coli der Patientin in Fläschchen mit physiologischer Lösung im Kühlschrank aufbewahrt.

Die Patientin führte eine Fastenkur und Diät durch:
- Gemüsediät in Kombination mit frisch gekochtem Brei aus Vollkorngetreide - 10 Tage; Entnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Abschlussphase - 1 Tag;
- vegetarische Ernährung - 10 Tage; Einnahme von typischen E.coli, E.coli laktose-negativ nach jeder Mahlzeit.

Der Gehalt an typischen und laktose-negativen E. coli war normal, und es gab keine hämolytischen Bazillen.

Während des Fastens war die Patientin in der Lage, einige Arbeiten auszuführen.

Die durch den Krebs verursachten Schmerzen gingen nach dem Kurs stetig zurück. Der Einsatz von Analgetika war nicht erforderlich. Bei den Mahlzeiten gab es keine Blähungen. Die Patientin hat die Fastenkur gut vertragen.

### Beispiel 3.

Patientin P., 29 Jahre alt, diagnostiziert mit übergewichtigem Dyspepsie-Syndrom.

Im Vorfeld des Kurses wurden traditionelle Fastenmethoden ohne Erfolg angewandt, Antazida, antimikrobielle Mittel und Prokinetika eingesetzt. Keine Resultate.

Nach den ersten Tests im biologischen Labor beträgt der Gehalt an typischen E.coli 2×10¹¹ KBE/g Kot, E.coli laktose-negativ < Zx10³ KBE/g Kot, E.coli hämolytisch -10³ .

Die typischen und laktose-negativen E.coli-Stämme der Patientin wurden zur Kultivierung entnommen. Während der Kultivierung wurden die E.coli der Patientin in Fläschchen mit physiologischer Lösung aufbewahrt und im Kühlschrank aufbewahrt.

Die wichtigste verordnete Fasten- und Diätkur für die Patientin umfasste:
- Gemüsediät in Kombination mit frisch gekochtem Brei aus Vollkorngetreide - 10 Tage; Entnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung - 10 Tage; Einnahme von typischen E.coli, E.coli laktose-negativ nach jeder Mahlzeit.

Typische und laktose-negative E.coli-Werte wichen geringfügig vom Normalwert ab, die Werte wurden durch Unterbrechung des E.coli-Kurses kontrolliert. Hämolytisch bei einer Konzentration von 500 cfu/r im Stuhl. Es wurde eine orale Rehydrierung verordnet, wobei der Patient bis zu 3 Liter Flüssigkeit pro Tag trinken sollte. Am Ende der Rehydratation wurden hämolytische E.coli - 0.

Während des Fastens konnte sie einige Arbeiten verrichten.

Als Ergebnis des Kurses und während der Hauptphase des Kurses hörten die durch abnorme Magentätigkeit verursachten Schmerzen auf, und die schwierige und schmerzhafte Verdauung hörte auf. Bei den Mahlzeiten gab es keine Blähungen. Die Patientin hat das Fasten gut vertragen.

### Beispiel 4.

Patientin G., 56 Jahre alt, Diagnose: chronischer Alkoholismus Grad II, charakteristische Schmerzen im rechten Subkostalbereich.

Vor der Behandlung unterzog sie sich einer Rehabilitationsmaßnahme, die u. a. Folgendes umfasste: Behandlung der durch den Rausch verursachten Störungen, Beseitigung der Folgen der Ethanolvergiftung, Behandlung der Alkoholsucht, Behandlung der durch Alkoholmissbrauch verursachten psychischen Störungen und Rehabilitation. Nach drei Monaten wurde er rückfällig, und die Schmerzen und das Verlangen nach Alkohol traten wieder auf.

Nach den ersten Tests im biologischen Labor ist der Gehalt an typischen E.coli 10⁷ CFU/r Faeces, E.coli laktosonegativ < 2×10⁴ CFU/g Faeces, E.coli hämolytisch - 0.

Die typischen und laktose-negativen E.coli-Stämme des Patienten wurden zur Kultivierung entnommen. Während der Kultivierung wurden die E.coli des Patienten in Fläschchen mit physiologischer Lösung aufbewahrt und im Kühlschrank aufbewahrt.

Die der Patientin verordnete grundlegende Fasten- und Diätkur umfasste:
- Gemüsediät in Kombination mit frisch gekochtem Brei aus Vollkorngetreide - 10 Tage; Entnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung - 10 Tage; Einnahme von typischen E.coli, E.coli laktose-negativ nach jeder Mahlzeit.
Der Gehalt an typischen und laktose-negativen E. coli war normal, und es gab keine hämolytischen Bazillen.

Während des Fastens konnte sie einige Arbeiten verrichten.

Am Ende des Kurses gab es keine Schmerzen im rechten Subkostalbereich und kein Verlangen nach Alkohol. Am Ende des Kurses zeigte eine Ultraschalluntersuchung der Leber eine Verbesserung des Zustands der Leber. Während der Mahlzeiten wurde kein Meteorismus beobachtet. Die Patientin hat die Fastenkur gut vertragen.

### Beispiel 5.

Patient D., 49 Jahre alt, diagnostiziert eine Nierensteinerkrankung.

Die Behandlung war konservativ und symptomatisch während der Nierenkolik: Heizkissen auf dem Nierenbereich, warme Bäder und parenterale Verabreichung von Drogen: 1 ml 2% Promedol, 1% Morphin, 1-2% Pantopon in Kombination mit Antispasmodika - 1 ml 0,1% Atropin und 1 mg 0, I % Platifyllin. Gelegentlich Wischnewski-Lumbalblock.

Die Nierenkoliken treten regelmäßig wieder auf.

Nach den Ergebnissen der ersten Tests im biologischen Labor beträgt der Gehalt an typischen E.coli 8×10⁷ CFU/g Faeces, E.coli laktose-negativ < 7×10⁴ CFU/g Faeces, E.coli hämolytisch - 0.

Die typischen und laktose-negativen E.coli-Stämme des Patienten wurden zur Kultivierung entnommen. Während der Kultivierung wurden die E.coli des Patienten in Fläschchen mit physiologischer Lösung aufbewahrt und im Kühlschrank aufbewahrt.

Die wichtigste verordnete Fasten- und Diätkur für den Patienten umfasste:
- Gemüsediät in Kombination mit frisch gekochtem Brei aus Vollkorngetreide - 10 Tage; Entnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung - 10 Tage; Einnahme von typischen E.coli, E.coli laktose-negativ nach jeder Mahlzeit.

Der Gehalt an typischen und laktose-negativen E. coli war normal, und es gab keine hämolytischen Bazillen.

Während des Fastens konnte er einige Arbeiten verrichten.

Nach den Ergebnissen des Kurses und während der Hauptphase hörten die durch die Nierenkolik verursachten Schmerzen auf, und sechs Monate lang, nachdem alle Nierensteine ausgespült worden waren, wurde kein Wiederauftreten beobachtet. Während der Mahlzeiten wurde kein Meteorismus beobachtet. Der Patient hat die Fastenkur gut vertragen.

Ohne ein Wiederauftreten abzuwarten, führte der Patient eine weitere Fastenkur nach der Methode durch, nach der ebenfalls sechs Monate lang keine Nierenkolik mehr auftrat.

Zusätzliche Fastenkurse werden alle sechs Monate empfohlen.

### Beispiel 6.

Bei Patientin K., 27 Jahre, wurde eine sekundäre Dystrophie auf dem Hintergrund einer Gastritis diagnostiziert.

Zusammen mit niedrigem Körpergewicht hatte er Lethargie und Apathie, trockene, schuppige Haut.

Übelkeit und Erbrechen wurden von starken Magenschmerzen begleitet. Eine Hintergrunderkrankung führte zu einer Pyelonephritis. Es gab ein Symptom der Dehydrierung in Form von Durst.

Vor dem Kurs fand die Behandlung im Krankenhaus statt, wo sie in der Korrektur der Ernährung (Diät), der Einnahme von Enzympräparaten und Multivitaminen sowie der Einnahme von antibakteriellen und antiviralen Medikamenten bestand.

Am Ende der stationären Behandlung kam es zu einem Rückfall.

Nach den ersten Tests im biologischen Labor liegt der Gehalt an typischen E.coli bei Z×10¹⁰ KBE/g Kot, E.coli laktose-negativ < 4×10¹¹ KBE/g Kot, E.coli hämolytisch - 0.

Die typischen und laktose-negativen E.coli-Stämme des Patienten wurden zur Kultivierung entnommen. Während der Kultivierung wurden die E.coli des Patienten in Fläschchen mit physiologischer Lösung im Kühlschrank aufbewahrt.

Die dem Patienten verordnete Hauptfasten- und Diätkur umfasste:
- pflanzliche Ernährung in Kombination mit frisch gekochtem Brei aus Vollkorngetreide - 10 Tage; Aufnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung - 14 Tage; Aufnahme von typischen E.coli, E.coli laktose-negativen Bazillen nach jeder Mahlzeit.

Typische und laktose-negative E.coli-Werte wichen geringfügig vom Normalwert ab, die Werte wurden durch Unterbrechung der E.coli-Versorgung kontrolliert. Es wurden keine hämolytischen Bazillen gefunden.

Während der Hauptphase des Fastens nahm das Durstgefühl ab und hörte am Ende des Fastens auf. Während der Fastenzeit konnte der Patient einige Arbeiten erledigen.

Am Ende der Fasten- und Diätkur, nach einer Woche normaler Lebensweise mit aktiver Ernährung, hörten die Schmerzen auf, die durch starke Magenschmerzen und Nierenkrämpfe verursacht wurden.

Die Lethargie, Apathie und schuppige Haut haben aufgehört.

Übelkeit und Erbrechen wurden sechs Monate lang nicht mehr beobachtet.

Während der Mahlzeiten wurden keine Blähungen beobachtet. Der Patient hat die Fastenkur gut vertragen.

Ohne ein Wiederauftreten abzuwarten, führte der Patient eine weitere Fastenkur nach der Methode durch, nach der ebenfalls sechs Monate lang keine Nierenkolik mehr auftrat.

Zusätzliche Fastenkurse werden alle sechs Monate empfohlen.

### Beispiel 7.

Patientin J., 57 Jahre alt, Diagnose: Magengeschwür. Schmerzhafte Symptome: Magenschmerzen nach dem Essen, Sodbrennen bis hin zum Erbrechen, schmerzhafte Verdauung im Zwölffingerdarm mit Krämpfen.

Vor dem Kurs wurde eine medikamentöse Behandlung durchgeführt: antisekretorische Mittel (H2-Histaminblocker, Na-K-ATPase-Blocker); Gastroprotektoren; Antazida; gastrointerstitielle Hormone.

Nach Abschluss der Behandlung kam es 2 Wochen später zu einem Rückfall der Krankheit in akuter Form.

Als konservative Maßnahme hat sie Almagel A verwendet, was zu schwerer Verstopfung führte.

Nach den ersten Tests im biologischen Labor beträgt der Gehalt an typischen E.coli 6×10⁸ CFU/g Faeces, E.coli laktose-negativ < 5×10⁵ CFU/g Faeces, E.coli hämolytisch - 0.

Die typischen und laktose-negativen E.coli-Stämme der Patientin wurden zur Kultivierung entnommen. Während der Kultivierung wurden die E.coli der Patientin in Fläschchen mit physiologischer Lösung im Kühlschrank aufbewahrt.

Die wichtigste verordnete Fasten- und Diätkur für die Patientin umfasste:
- Gemüsediät in Kombination mit frisch gekochtem Brei aus Vollkorngetreide - 10 Tage; Entnahme von typischen E.coli, E.coli laktose-negativen Bazillen nach jeder Mahlzeit;
- Fastenkurs (nach der Entleerung):
- der Grundzyklus des Fastens:
- Anfangsphase -1 Tag;
- Hauptphase -11 Tage;
- Schlussphase -1 Tag;
- vegetarische Ernährung -10 Tage; Aufnahme von typischen E.coli, E.coli laktose-negativ nach jeder Mahlzeit.

Typische und laktose-negative E.coli-Werte wichen geringfügig vom Normalwert ab, die Werte wurden durch Unterbrechung des E.coli-Kurses kontrolliert. Hämolytische Bazillen waren nicht vorhanden.

Nach den Ergebnissen des Kurses und während seiner Hauptphase hörten die durch Magengeschwüre und Sodbrennen verursachten Schmerzen auf, und die schmerzhafte Verdauung im Zwölffingerdarm hörte auf.

Sechs Monate lang gab es keine Rückfälle. Während des Fastens war sie in der Lage, einige Arbeiten auszuführen.

Während der Mahlzeiten wurden keine Blähungen beobachtet. Die Patientin hat die Fastenkur gut vertragen.

Ohne das Auftreten von Rückfällen abzuwarten, führte die Patientin eine weitere Fastenkur nach der Methode durch, nach der ebenfalls sechs Monate lang kein Rückfall auftrat.

Regelmäßige zusätzliche Fastenkurse alle sechs Monate werden empfohlen.

Einige der oben genannten Patienten erhielten zusätzliche 3-8 Rehabilitationskurse mit der vorgeschlagenen Methode, mit der weiteren Empfehlung, diese Technik zweimal im Jahr als Prophylaxe anzuwenden.

Die beanspruchte Methode erleichtert es den Patienten, eine Fastenkur mit einem verminderten Durstgefühl und einem verminderten Schmerzempfinden zu ertragen, und ermöglicht es ihnen, während des Fastens bestimmte Arbeiten auszuführen.

## Patentansprüche

1. Die Erfindung betrifft eine Fastenmethode für schwerkranke Patienten, bei der auf Nahrung und Flüssigkeit verzichtet wird und eine vegetarische Diät mit einer pflanzlichen Diät oder einer pflanzlichen Diät in Kombination mit frisch gekochtem Vollkorngetreide 5 bis 10 Tage vor Beginn des Fastens durchgeführt wird. Das Fasten beginnt nach der Darmentleerung und besteht aus einem Grundzyklus, der aus einer ungeteilten Anfangs-, Haupt- und Schlussphase besteht. Die Anfangsphase dauert 1 bis 3 Tage und schließt Nahrung und Flüssigkeiten außer Wasser und Urin aus. Die Hauptphase dauert mindestens 11 Tage mit vollständigem Verzicht auf Nahrung und Flüssigkeit. Die letzte Phase dauert 1-3 Tage, in denen keine Nahrung und keine Flüssigkeiten außer Wasser und Urin aufgenommen werden dürfen. Gleichzeitig wird in der Hauptphase des Hauptfastenzyklus eine Mundspülung durchgeführt. ***Der Unterschied besteht darin,*** dass dem Patienten zuvor lebende Stämme typischer E.coli und laktose-negativer E.coli entnommen werden, um ihre Kultur unter künstlichen Bedingungen zu züchten. Die Bakterienkulturen werden für die Dauer des Fastens aufbewahrt, und nach der letzten Phase des Fastens werden die Kulturen typischer und laktose-negativer E.coli des Patientenstammes während einer vegetarischen Diät wieder verabreicht. Nach dem ersten Stuhlgang wird der Patient auf normale E.coli-Werte getestet. Folgende Werte gelten als normal: typische E.coli 10⁷ -10⁸ KBE/g Fäkalien, E.coli laktose-negativ < 10⁵ KBE/g Fäkalien, E.coli hämolytisch - 0. Bei abnormalen E.coli-Werten werden die entsprechenden typischen und/oder laktosonegativen E.coli-Stämme erneut verabreicht. Bei überhöhten E.coli-Werten werden die entsprechenden typischen und/oder laktosonegativen E.coli von der nächsten Mahlzeit ausgeschlossen. Wenn hämolytische E.coli nachgewiesen werden, wird eine orale Rehydratation verordnet, bei der der Patient bis zu 3 Liter Flüssigkeit pro Tag trinken soll.

2. Die Vorgehensweise gemäß P. 1, **mit dem Unterschied,** dass am Ende des Fastens eine 10-tägige vegetarische Diät verordnet wird, die eine pflanzliche Diät oder eine pflanzliche Diät in Kombination mit frisch gekochten Breien aus ganzen Getreidekörnern ist, und für Patienten mit Anzeichen von Dystrophie eine vegetarische Diät mit einer Diätdauer von bis zu 14 Tagen verordnet wird.

3. Die Vorgehensweise gemäß P. 1, mit **dem Unterschied,** dass die Aufnahme von kaliumreichen Lebensmitteln verordnet wird.

4. Die Vorgehensweise gemäß P. 1, **mit dem Unterschied,** dass 250 g Kaogsil drei Wochen lang zwischen den Fastenkuren verordnet wird.

5. Die Vorgehensweise gemäß P. 1, **mit dem Unterschied,** dass die Hauptphase des Grundfastenzyklus 22 Tage lang dauert.

6. Die Vorgehensweise gemäß P. 1, **mit dem Unterschied,** dass eine Fastenkur mindestens einen zusätzlichen Fastenzyklus umfasst, der vom Hauptzyklus oder von einem anderen zusätzlichen Fastenzyklus durch eine vegetarische Ernährung getrennt ist. Jeder zusätzliche Fastenzyklus hat eine ungeteilte, aufeinander folgende Anfangs-, Haupt- und Endphase. Die Anfangsphase dauert 1 bis 3 Tage, in denen keine Nahrung oder Flüssigkeit außer Wasser und Urin aufgenommen wird. Die Hauptphase dauert weniger als 11 Tage und umfasst den vollständigen Verzicht auf Nahrung und Flüssigkeit. Die letzte Phase dauert 1-3 Tage, in denen Nahrung und Flüssigkeit außer Wasser und Urin ausgeschieden werden. Während der Hauptphase eines jeden zusätzlichen Fastenzyklus wird eine Mundspülung durchgeführt.

7. Die Vorgehensweise gemäß P. 1, **mit dem Unterschied,** dass die Mundspülung während des Fastens mit Wasser oder leichten Getränken durchgeführt wird.

8. Die Vorgehensweise gemäß P. 1, **mit dem Unterschied,** dass die Dauer der vegetarischen Diät zwischen zwei zusätzlichen Fastenzyklen oder zwischen dem zusätzlichen Fastenzyklus und dem Hauptfastenzyklus ein bis zehn Tage beträgt, wobei die Dauer der vegetarischen Diät zwischen zwei zusätzlichen Fastenzyklen gleich der Dauer der Hauptfastenphase des vorhergehenden zusätzlichen Fastenzyklus ist.

9. Die Vorgehensweise gemäß einem der P. 1 bis 8, **mit dem Unterschied,** dass nach einer Fastenkur weiße Tonerde mit Diät verabreicht wird.
